(19) Europäisches Patentamt
European Patent Office
Office européen des brevets

(11) **EP 2 103 279 B1**

(12) **EUROPEAN PATENT SPECIFICATION**

(45) Date of publication and mention
of the grant of the patent:
**24.08.2011 Bulletin 2011/34**

(51) Int Cl.:
***A61F 2/16*** (2006.01)

(21) Application number: **09155606.8**

(22) Date of filing: **19.03.2009**

(54) **Multifocal intraocular lens**

Multifokale Intraokularlinse

Lentille intraoculaire multifocale

(84) Designated Contracting States:
**AT BE BG CH CY CZ DE DK EE ES FI FR GB GR
HR HU IE IS IT LI LT LU LV MC MK MT NL NO PL
PT RO SE SI SK TR**

(30) Priority: **19.03.2008 DE 202008003859 U**

(43) Date of publication of application:
**23.09.2009 Bulletin 2009/39**

(73) Proprietor: **Icon Lab GmbH
51702 Bergneustadt (DE)**

(72) Inventors:
• **Treushnikov, Valeriy
603139, Nizhniy Novgorod (RU)**

• **Cherednik, Valentin
603098, Nizhniy Novgorod (RU)**
• **Viktorova, Elena
603104, Nizhniy Novgorod (RU)**
• **Starostina, Olga
603139, Nizhniy Novgorod (RU)**

(74) Representative: **Lippert, Stachow & Partner
Patentanwälte
Frankenforster Strasse 135-137
51427 Bergisch Gladbach (DE)**

(56) References cited:
**EP-A- 1 891 912          WO-A-2006/023404
US-A1- 2004 252 274     US-A1- 2007 216 851**

## Description

The present invention relates to medicine, or, to be more specific, to ophthalmology, and is intended for eyesight correction by means of implantation in an mammal eye, especially human eye.

It is known that there exist hybrid type multifocal lenses, which have both a refractive and a diffractive part (Patents US 4,637,697 MULTIFOCAL CONTACT LENSES UTILIZING DIFFRACTION AND REFRACTION; WO/2004/113959 BIFOCAL MULTIORDER DIFFRACTIVE LENSES FOR VISION CORRECTION; US5089023 DIFFRACTIVE/REFRACTIVE LENS IMPLANT; US7025456 DIFFRACTIVE LENSES FOR VISION CORRECTION; RU 2303961 - MULTIFOCAL INTRAOCULAR LENS; US 5,344,447 DIFFRACTIVE TRIFOCAL INTRAOCULAR LENS DESIGN (prototype). The drawback of this invention (as well as of all diffractive-refractive intraocular lenses (IOL), irrespective of their design features) lies in the fact that the thickness of the hybrid IOLs of this type do not differ from and therefore cannot be smaller than the thickness of the ordinary monofocal refractive IOLs, both planoconvex and biconvex refractive lens, on one of which there is a diffractive microstructure in the form of rings, the radii of which coincide with the radii of the Fresnel zones. Then in the case of such IOL type the refractive component of the lens has the optical power D calculated for distance vision, that is in the same way as for ordinary monofocal IOL. The additional optical power of the diffractive component is made to be not more than $\Delta D = 3\text{-}4$ dioptres in order to provide for the accommodation depth necessary for near vision at a distance of 25-35 cm. In case of diffractive-refractive IOL the light flux is directed into two focuses, for distance vision (the optical power equals D) and for near vision (the optical power equals $D+\Delta D$). A reduction in traumatism of the eye in the process of cataract extraction with IOL implantation in actual fact can be efficiently achieved only by way of using smaller and smaller incisions - less than 1.5 mm. At present time there are phacoemulsificators that make it possible to do cataract extraction through such small incisions, but there are no (and there cannot be any) refractive IOLs that could be implanted through the small size incisions, including manufacture of diffractive-refractive IOL under all the above mentioned patents. In fact, this creates a ban (a barrier) for utilizing this principle to create multifocal (pseudo-accomodating) IOLs designed for implantation through super small incisions.

The proposed invention accomplishes the object to reduce eye traumatism during the surgery, to shorten post-surgical time period, to increase in visual acuity, and to ensure of a constant quality image at any distances from the objects.

This object is solved by a multifocal intraocular lens (IOL) according to claim 1. Unlike in known solutions the diffractive component of such IOL is much bigger than the known ones have, and together with refractive part it ensures light entry into one focus - for distance vision. For example, if optical power of refractive part of IOL equals to 10 dioptres, diffractive part equals 10 dioptres as well, then optical power of that IOL equals to 20 dioptres . Such IOL will have thickness that is half as large as an ordinary refractive lens with the same optical power. Separation of light energy between focuses for distance vision and near vision is carried out on account of creation of inside refractive surfaces, which divide optical part of IOL into zones made of different materials with different refraction coefficients.

Preferred embodiments are described in the subclaims.

The technical result of using the invention enables to make multifocal (pseudo-accommodating) IOLs with the thicknesses approximately two times smaller than those of ordinary (refractive) monofocal IOLs and of all hybrid, diffractive-refractive IOLs.

According to the invention the proposed multifocal intraocular lens comprises two external refractive surfaces, on one of which there is a diffractive structure in the form of rings, and between the external refractive surfaces additional refractive surfaces are inserted dividing the lens volume into zones made of materials with different refraction coefficients. Each zone may have a refraction coefficient being different from any other refraction coefficient of any other zone.

The zones may be arranged one after the other generating at least substantially parallel layers made of different materials which differ in refraction coefficient in such a manner that centres of all surfaces coincide with optical axis of the lens.

One of the two external refractive surfaces of the lens may be a sphere, preferably a radial sphere, and the other one may be a plane with a diffractive structure in the form of rings superimposed all over its surface, the radii of the rings coinciding with the radii of the Fresnel zones. Between the two external refractive surfaces there is exactly one surface dividing the volume of the lens into two zones made of two different materials with different refraction coefficients according to one embodiment.

According to another preferred embodiment there are two surfaces dividing the volume of the lens into three zones made of three different materials with different refraction coefficients, wherein adjacent zones may have different refraction coefficients or each zone may have a refraction coefficient being different from the refraction coefficient of any other zone. In principle, there may be a number m of surfaces dividing the volume of the lens into a number of m + 1 zones, with $m \geq 2$.

The difference in the refraction coefficients of the different materials used to make the lens should not be $\leq 0.02$. at the same time all the materials must have refraction coefficient that is not less than 0.02 bigger than refraction coefficient of ocular fluid. That is, the upper limit of materials refraction coefficient should not be less than 1.336 + 0.02 = 1.356 (1.336 is the refraction coefficient of ocular liquid according to reference data). In theory, the maximum difference may compose up to 0.64 (if refraction coefficient of the material equals to 2.0, then 2.0-1.356=0.64. Meanwhile, materials with maximum refraction coefficient of 1.55 is used. That is, the actual maximum difference comprises 0.194). Herein it is better not to use specific figures but to specify an interval in terms of dependence on refraction coefficient of material used. So, let's make identifications, n is a refraction coefficient of ocular fluid, n1 is a refraction coefficient of external refractive surface (refractive-diffractive), n3 is a refraction coefficient of the second internal additional refractive surface. Then the refraction coefficient of the material of the first internal additional refractive surface lies in the interval n+0.02<n2<n1 -0.02, the material refraction coefficient of the second internal additional refractive surface lies in the interval n1+0.04<n3<n2-0.02.

The diffractive structure in the form of rings, the radii of which coincide with the radii of the Fresnel zones, is preferably manufactured in such a way, preferably based on appropriate calculation, that the additional optical power of this structure is preferably ensured within the interval of 8 to 12 dioptres.

The statistical average for the human eye refraction is 20-22 D (dioptres). The additional optical power of the diffractive part being from 8 to 12 D makes it possible to decrease the thickness of the lens significantly, e.g. by half or in a corresponding different magnitude depending on the additional optical power.

The microrelief of the diffractive structure may must be in the form of at least substantially right-angled profile grooves for every only even or only odd Fresnel zones with the depth $h = \dfrac{0{,}55}{2(n_1 - n)}$ micrometers, with fractional accuracy of deviation to be not more than 5%

Alternatively, the microrelief of the diffractive structure may be at least substantially in the form of triangle profile grooves uniting every two neighbouring Fresnel zones with the height of the triangle $h = \dfrac{0{,}55}{(n_1 - n)}$ micrometers with fractional accuracy of deviation to be not more than 5 %. Where n1 in either case is the refraction coefficient of the lens zone, which has the diffractive structure on it, n is the index of refraction of ocular fluid.

In certain cases, the microrelief structure may be in the form of or may comprise grooves having cross section of intricate profile, for example, along the line of the sinusoidal function.

The triangle profile lens may have two diffraction maximums - the zeroth-order diffraction maximum (0) and of the plus first-order diffraction maximum (+1). The right-angled profile lens may have three diffraction maximums - the zeroth-order diffraction maximum (0), the plus first-order diffraction maximum (+1) and the minus first-order diffraction maximum (-1), Varying the depth of the microrelief makes it possible to alter power distribution among the diffraction foci of the lens. According to this invention the depths of the grooves are selected in such a manner that in case with the triangle profile all the energy would be concentrated only in the first order (+1) maximum, and with the right-angled profile it would be concentrated in the plus first-order (+1) and minus first-order (-1) diffraction maximums. In the case with the right-angled profile when image formation on the retina only the plus first-order maximum (+1) plays the main part; the minus first-order maximum (-1) doesn't influence the image quality due to long distance from the plus first-order maximum (+1). The Fresnel zone radii, calculated without taking into account the spherical aberration of the optical system of the eye, are directly proportional to the square roots of the integers designating the Fresnel zone's index number rk =r1 √k, where k= 1,2,3..., r1 is the radius of the first Fresnel zone calculated in such a way that the prescribed optical power of the diffractive structure is ensured within the interval of 8 to 12 dioptres. When calculating the radii of the Fresnel zones according to the formula rk= r1 √k, where k= 1,2,3..., the spherical aberration of the optical system of the eye affects the image quality insignificantly, only when the diffractive structure is superimposed not all over the entire external plane surface of the lens, but only in its central part. The radii of the Fresnel zones superimposed all over the external plane surface of the lens should be calculated taking into account the spherical aberration of the entire optical system of the eye.

This kind of calculation can only be done with, the help of known procedures of computer modelling of the entire optical system of the eye, which makes it possible to reduce or to minimize the spherical aberration of the optical system of the eye, including the cornea and all the refractive surfaces of the crystalline lens.

The IOL may contain either one additional refractive surface in its central part, which is represented by a spherical segment with the diameter d1 within the range of 1.6 to 2.6 mm, or 1.7 to 2.5 mm, preferably 1.8 to 2.4 mm or 2.0 to 2.2 mm, further changing radially outwardly into a plane. Such construction of the additional refractive surface allows to avoid undesirable optical effects, such as 'halo' with any intensity of the light on account of 'superfluous' light that appears on the boundary line of two zones with different refractive coefficients, and goes beyond the circumference of the lens. Alternatively, two additional refractive surfaces may be provided, the first of which, counting form the external surface of the lens with the diffractive structure, is located in its central part and is represented by a spherical segment with the diameter d2 within the range of 1.4 to 1.8 mm, or 1.5 to 1.7 mm, further changing into a plane, and the second additional surface in its central part is represented by a spherical segment with the diameter d3 within the range of 2.1 to 2.6 mm or 2.2 to 2.5 mm or 2.3 to 2.4 mm into a plane.

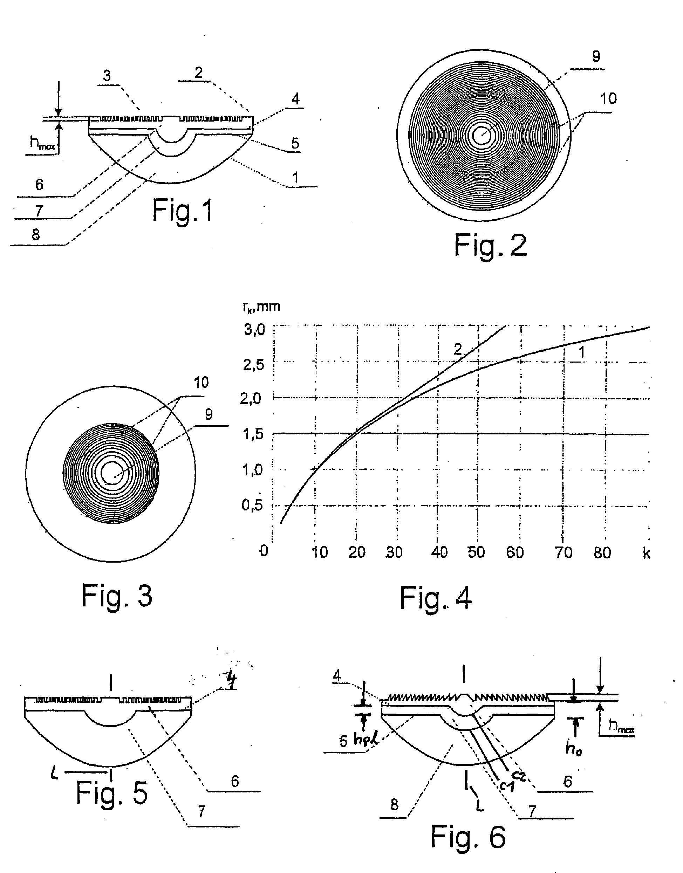

The thickness of the planes hpl (Fig.6) mentioned above may be in the region of 25 to 150 micrometers or 50 to 100 micrometers. The thickness of the planes hpl may be the thickness of the flat end of the internal lens. The thickness of the planes doesn't influence the image quality. In the optical part the thickness may be variable, determined by the refraction needed for each patient.

The total thickness of the end face of the lens h0 may be 200-250 micrometers (Fig. 6), in some instances, less preferred, 100-500 micrometers or 150-350 micrometers. Thickness h0 may refer to the overall thickness of the flat end of the lens without taking into consideration hmax, i.e. the thickness of the diffractive microrelief. Smaller thickness doesn't allow to place all the planes of additional refractive surfaces inside the lens, generated by two external reflective surfaces, and bigger thickness of the end face of the lens leads to the complication of incurvation and implantation into the eye of a patient.

The thickness of the convex portion of the lens depends on the radii of its spherical surface, which in its turn is calculated in such a way that with target refractive coefficients of internal surfaces to provide partition of light energy between focuses with difference of 3-4 dioptres.

The multifocal lens according to the invention may be used in other fields than surgery, if appropriate, e.g. in systems wherein the lens is immersed or embedded with one or both external refractive surfaces in a fluid, wherein the fluid preferably may have a refractive coefficient like water (pure water) or aqueous solutions, including solutions having a refractive coefficient about 1.28 to 1.4, especially 1.30 to 1.37, most preferably about or equal 1.336.

The essence of the invention is explained below with reference to the accompanying drawings, in which:

Fig. 1 is a diagram showing the optical part of the lens with the right-angled profile diffractive structure and two additional refractive surfaces dividing the volume of the lens into three zones made of three different materials having different refraction coefficients n1, n2, n3.
1 - the external refractive surface of the lens; 2 - the external refractive surface with a diffractive structure;3 - the microrelief of the right-angled profile diffractive structure; 4 - the first internal refractive surface;. 5 -the second internal refractive surface; 6 - the zone with the-refraction, index of the material n1; 7 - the zone with the refraction index of the material n2, 8 - the zone with the refraction, index of the material n3; hmax-the diffractive structure microrelief depth.

Fig. 2 is a diagram showing the ring-type diffraction zones on the plane surface of the lens, calculated with the help of computer modelling, taking into account the spherical aberration of the optical system of the eye, superimposed all over the external plane surface of the lens.
9-the central ring-zone with a radius r1; 10-concentric ring-type diffraction zones with radii r2....rk.

Fig, 3 is a diagram showing the ring-type diffraction zones on the plane surface of the lens, calculated according to the formula rk =r1 √k, where k = 1,2,3..., superimposed on the central part of the plane surface of the lens to minimize the spherical aberration.

Fig. 4 is a graphical representation of the dependency of the diffractive structure rings radii on their numbers: curve 1 represents the Fresnel zones' radii calculated according to the formula rk =r1 √k, where k = 1,2,3...; curve 2 represents the Fresnel zones' radii calculated with the help of computer modelling, taking into account the spherical aberration of the optical system of the eye.

Fig. 5 is a diagram showing the lens with the right-angled profile diffractive structure and one additional refractive surface (4) dividing the lens volume into two zones (6 and 7) made of two different materials having different refraction coefficients n1 and n2, respectively.

Fig. 6 is a diagram showing the lens with the triangle profile diffractive structure and two additional refractive surfaces (4,5) dividing the lens volume into three zones (6,7,8) made of three different materials with different refraction coefficients n1, n2, and n3, respectively.

Fig. 7 is a graphical representation of the light intensity distribution produced by the optical system of the eye with a bifocal diffractive-refractive lens with the right-angled profile of microrelief.

Fig. 8 is a diagram showing the additional refractive surface in the form of a spherical segment with the diameter d1 within the range of preferably 2.0 to 2.2 mm, further changing into a plane.

Fig. 9 is a diagram showing two additional refractive surfaces, the first of-which, counting from the external surface of the lens with the diffractive structure, is located in the central part and is represented by a spherical segment with the diameter d2 within the range of preferably 1.7 to 1.8 mm, further changing into a plane, and the second additional surface in the central part of the lens is represented by a spherical segment with the diameter d3 within the range of preferably 2.4 to 2.5 mm, further changing into a plane.

A proposed intraocular lens variant is depicted in Fig. 1.

The lens has a plano-convex shape formed by two external refractive surfaces, one of which is represented by a sphere (1), and the other one is represented by a plane (2) with a diffractive structure microrelief in the form of rings superimposed all over its surface, the radii of these rings coinciding with the radii of the Fresnel zones (3), inside the lens there is one (4) or two refractive surfaces (4, 5) represented by spheres. The external refractive surface, represented by a sphere, creates the main optical power by means of refraction phenomena. The additional optical power is provided for by means of diffraction on the diffractive structure microrelief (3) and refraction on one or two internal surfaces (4, 5). The microrelief is superimposed on the plane surface of the lens (2) in such a way that ring-type diffractive zones are formed on its surface (Fig. 2): the central zone (9) having the radius n and the ring-type concentric zones (10) with the radii r2,...rk. The Fresnel zones' radii depicted in Fig. 2 have been calculated with the help of computer modelling, taking into account the spherical aberration of the optical system of the eye, in such a way that the prescribed optical power of the diffractive structure is ensured within the interval of 8 to 12 dioptres. The statistical average for the human eye refraction is 20-22 D (dioptres). The optical power of the diffractive part being 8 to 12 D makes it possible to decrease the thickness of the lens approximately by half. The diffractive structure, similar to the one depicted in Fig. 2, provides for additional optical power of 10 dioptres on condition that the radius of the first ring-type Fresnel zone r1 = 0.25 mm.

The number and positioning of the diffraction zones depend on the needed value of the additional optical power that the lens needs to provide, the diameter of the lens, the light wave length and the influence degree of the spherical aberration of the optical system of the eye. The proposed lens variant depicted in Fig. 2 and the variant depicted in Fig. 3 differ from each other in the ways of minimizing the influence on the diffraction image of the spherical aberration of the optical system of the eye. The lens proposed in Fig. 2 has the diffractive structure superimposed on almost its entire plane surface. The elimination of the spherical aberration's influence is achieved, in this case, by selecting, with the help of computer modelling, a special law regulating the dependency of the diffractive relief rings radii on the rings' numbers.

The lens proposed in Fig. 3 has the diffractive structure superimposed on just the central part of the plane surface of the lens. This kind of the proposed lens's design makes it possible to minimize the spherical aberration's influence on the diffraction image. This is illustrated by Fig. 4, which shows the dependencies of the rings' radii on their numbers, calculated both according to the formula rk = r1 √k (curve 1) and with the help of computer modelling, taking into account the spherical aberration (curve 2).

In Fig. 4 it is evident that in the central part of the lens, where the spherical aberration is small, both of the curves almost coincide, if the diffractive relief (3) is superimposed only on the central part of the plane surface of the lens, then the spherical aberration's influence on the diffraction image will be insignificant. The design in Fig. 3 actualizes this very way of minimizing the spherical aberration's influence on the diffraction image.

One of the variants of the proposed lens has the right-angled profile of the diffractive structure (Fig. 5).

A lens with the right-angled profile of the diffractive structure without any additional refractive surfaces provides for three diffraction maximums - the plus first-order diffraction maximum (+1), the zeroth-order diffraction maximum (0), and the minus first-order diffraction maximum (-1).

Another variant of the proposed lens has the triangle profile of the diffractive structure (Fig. 6).

A lens with the triangle profile of the diffractive structure without any additional refractive surfaces provides for two diffraction maximums - the plus first-order diffraction maximum (+1) and the zeroth-order diffraction maximum (0).

The power distribution among the diffraction maximums may vary. The power distribution is influenced by the depth of the diffractive structure microrelief hmax (Fig. 6).

The depth of the right-angled diffractive structure microrelief is determined with the help of computer modelling in such a way that the intensity of the plus first-order (+1) diffraction maximum and of the minus first-order (-1) diffraction maximum be at their maximum levels, and the intensity of the zeroth-order (0) diffraction maximum be equal to zero. With the depth of the right-angled profile microrelief calculated according to the formula

$$hMAX = \frac{0,55}{2(n1 - n)}$$

micrometers (n1 is the refraction index of the lens zone that has the diffractive structure on it, n is the refraction index of ocular fluid equal to 1.336), the minus first-order (-1) diffraction maximum is located beyond the retina and is not involved in the image formation, the intensity of the zeroth-order (0) diffraction maximum with the calculated microrelief depth equals to zero, so this maximum does not influence the quality of the image formed by the lens either, only the plus first-order (+1) diffraction maximum participates in forming the image on the retina. For the proposed lens the microrelief depth constitutes 1.65 micrometers

The depth of the triangle profile of the diffractive-structure microrelief for the proposed lens is calculated according to the formula

$$hMAX = \frac{0,55}{(n1 - n)}$$

micrometers {n1 the refraction index of the lens zone, which has the diffractive structure on it, n is the refraction index of ocular fluid equal to 1.336) (Fig. 6). With the calculated microrelief depth, the intensity of the zeroth-order (0) diffraction maximum equals to zero, and this maximum does not influence the quality of the image formed by the lens, practically all of the power is concentrated within the plus first-order (+1) diffraction maximum actually forming the image on the retina. For the proposed lens the triangle profile microrelief depth constitutes 3.3 micrometers,

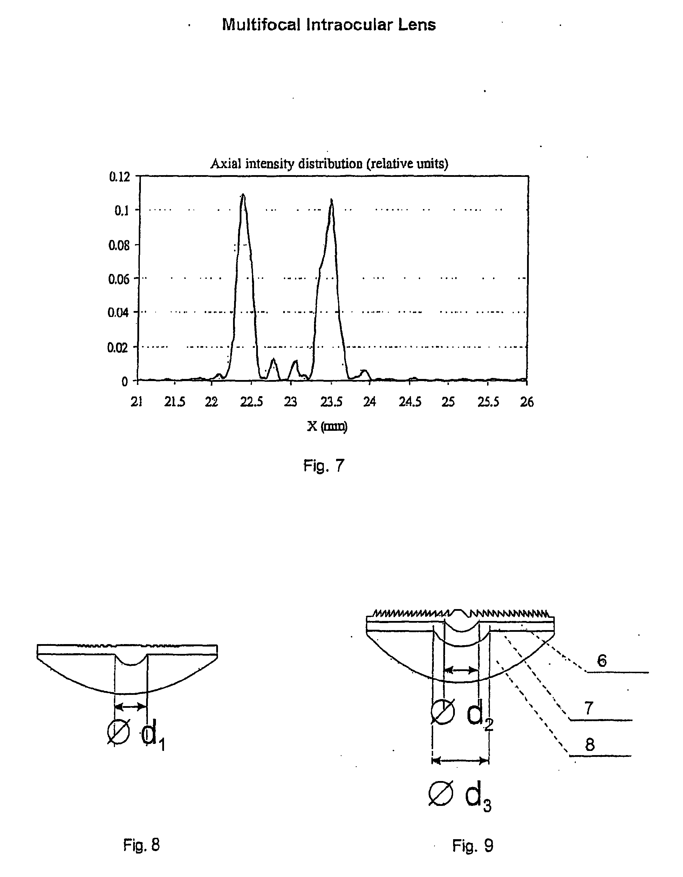

In one of its variants (Fig. 5) the proposed lens has one additional internal refractive surface (4), which divides the lens volume into two zones (6) and (7) made of materials with different refraction coefficients m, n2, with a right-angled profile microrelief on the plane surface of the lens. In this case each diffraction maximum bifurcates due to the additional refractive surface in the central part of the lens. One part of the light flux going though the central part of the lens goes through two spherical refractive surfaces and forms diffraction maximums in one set of places on the longitudinal axis L, the other part of the light flux, going through the peripheral part of the lens, encounters on its way only one external spherical refractive surface and forms diffraction maximums in another set of places on the longitudinal axis L. Thus, the said lens variant provides for bifocal vision by way of using the plus first-order (+1) bifurcated diffraction maximum. For example, the axial light intensity distribution for this variant, received with the help of computer modelling of the optical system of the eye, is depicted in Fig. 7. In particular, in Fig. 7 one can see that on the retina (the retina coordinate is 23.5 mm as related to the frontal surface of the cornea) there is one of the two diffraction maximums of the plus first-order (+1) that provides for a sharp image of distant objects, the zeroth-order maximum is completely suppressed by means of the microrelief depth that has been selected, the two minus first-order (-1) maximums are far beyond the retina and beyond the drawing in Fig. 7. Besides, in Fig. 7 one can see that the plus first-order diffraction maximum is divided into two approximately equal intensity maximums. This division is conditioned by the influence of the additional internal refractive surface (4), because of which both the central and the peripheral parts of the said lens focus light in two different points on the optical axis. The second maximum provides for the near vision (at a 30-33 cm distance).

In the other variant (Fig. 1) the proposed lens has two additional internal refractive surfaces (4, 5) that divide the lens volume into three zones (6, 7, 8) made of materials with different refraction coefficients n2, n3, with the right-angled profile microrelief on the plane surface of the lens. This variant of the lens provides for trifocal vision due to the fact that the plus first-order (+1) diffraction maximum is divided into three approximately equal intensity maximums. This division is conditioned by the influence of the two additional internal refractive surfaces (4, 5), because of which both the middle and the peripheral parts of the said lens focus light in three different points on the optical axis

Bifocal and trifocal vision can also be provided for by the proposed lens variants with the triangle profile of the diffraction relief. A lens, similar to the one in Fig.

5, but with a triangle relief of the diffraction profile, provides for bifocal vision by means of the bifurcated plus first-order (+1) diffraction maximum, too. This bifurcation is conditioned by the influence of the additional internal refractive surface (4), because of which both the central and the peripheral parts of the said lens focus light in two different points on the optical axis. The zeroth-order diffraction maximum is completely suppressed in this case, due to the selected depth of the triangle profile diffraction relief grooves.

In the other variant, the proposed lens (Fig. 6) has two additional internal refractive surfaces (4, 5) that divide the lens volume into three zones (6, 7, 8) made of materials with different refraction coefficients n, n2, n3, with the triangle profile microrelief on the plane surface of the lens. This variant of the lens provides for trifocal vision due to the fact that the plus first-order (+1) diffraction maximum is divided into three approximately equal intensity maximums. This division is conditioned by the influence of the two additional internal refractive surfaces (4, 5), because of which both the middle and the peripheral parts of the said lens focus light in three different points on the optical axis. The zeroth-order diffraction maximum is completely suppressed in this case, due to the selected depth of the diffraction profile microrelief.

In general, independent from the embodiment of fig. 6 and independent from the microrelief structure, the curvature c1 (i.e. radius of curvature) of the first internal refractive surface 5 may be larger than the curvature c2 (i.e. radius of curvature) of the second internal refractive surface 4, i.e. in either case the curvature in the plane of the drawing of fig. 6 as shown (paper plane) being perpendicular to the external front surface of the lens 2. This may hold especially in a region at or close to the optical axis (i.e. longitudinal axis L) of the lens or at height of central zone 9. In some instances, depending on the desired optical properties of the lens, the curvature c2 of an internal refractive surface may be smaller than the curvature c1. In general, this relation may be given referring to each pair of surfaces being adjacent in the longitudinal axis, if the lens comprises more than one internal zones.

The proposed lens contains one additional refractive surface in the central part of the lens, which is represented by the spherical segment with the diameter d1 within the range of 2,0 to 2.2 mm, further changing into a plane (Fig. 8). In humans the pupil diameter depends on the intensity of light entering the eye - the higher the intensity of light, the smaller is the diameter of the pupil. In a healthy human eye the minimum diameter of the pupil is approximately 3.0 mm, the maximum diameter is approximately 6.0 mm. If d1>3.0 mm, then in bright light (minimum pupil diameter) the human being will not be able to see objects clearly either at long or at short distances, depending on the implanted IOL type. The solution in the proposed lens consists in the fact that d1 is within the range of 2.0 to 2.2 mm. With d1 ≈2,0 mm and in bright light (pupil diameter - 3 mm) the light energy entering the eye is approximately equally divided between the two foci.

In the other variant (Fig. 9) the proposed lens contains two additional refractive surfaces, the first of which, counting from the lens' external surface with the diffractive structure, is located in the central part and is represented by the spherical segment with the diameter d2 within the range of 1.7 to 1.8 mm, further changing into-a plane, and the second additional surface in the central part is represented by the spherical segment with the diameter d3 within the range of 2.4 to 2.5 mm, further changing into a plane (Fig. 9).

The method of manufacture of the proposed multifocal intraocular lens (Fig. 6) with two external refractive surfaces, on one of which there has been superimposed a diffractive structure in the form of rings, the radii of which coincide with the radii of the Fresnel zones, and between its external refractive surfaces additional refractive surfaces have been inserted, that divide the lens volume into zones manufactured from materials having different refraction coefficients" comprises the optical part formation by way of using different photocurable materials with refraction indices n, n2, n3, their casting, UV treatment and removal of the uncured material, all this done consecutively in several stages using quartz casting mould assemblies. The quartz casting moulds consist of interchangeable halves, on the work surface of one of which there is a relief presetting the external refractive surface, and on the other one there is a diffractive structure in the form of rings, the radii of which coincide with the radii of the Fresnel zones, the other halves have work surfaces, on which the internal refractive surfaces of the lens are formed that have spherical holes with the diameter either d1 or d2 or d3 further changing into planes, additionally, on the work surface of the form half there is a pattern corresponding to the haptical part of the lens.

The first stage is the formation of the lens component representing the lens zone (8) restricted by the external refractive surface (1) and the first internal refractive surface (5) made of a photocurable material with the refraction index n3. The casting mould is assembled from two halves, the first of which presets the form of the external refractive surface of the lens (1), and the second one presets the form of the first internal refractive surface of the lens (5). The material is photocured by UV light, the two halves of the casting mould are divided in such a way that the resultant component stays on that half, which forms the external refractive surface of the lens (1), the uncured material is removed from the surface (5) of the resultant component with the help of an appropriate solvent - isopropyl alcohol, for instance, - and is dried till the solvent is gone.

The second stage is the formation of the lens component representing the lens zone (7) restricted by the first internal refractive surface (5) and the second internal refractive surface (4), made of a photocurable material with the refraction index n2. The manufacturer takes the half of the casting mould with the lens zone formed on it during the first stage (8), casts the photocurable material with the refraction index n2 and closes it with the other half that presets the form of the second internal refractive surface of the lens (4). The material is photocured by UV light, the two halves of the casting mould are divided in such a way that the resultant component - zone (7) - stays on that half of the mould, on which a zone has already been formed (8), the uncured material is removed from the surface (4) of the resultant component with the help of an appropriate solvent - isopropyl alcohol, for instance, - and is dried till the solvent is gone.

The third stage is the formation of the lens component representing the lens zone (.6); restricted by the external refractive surface with the diffractive structure in the form of rings, the radii of which coincide with the radii of the Fresnel zones (2). The manufacturer takes the half of the casting mould with the lens zone formed on it during the first stage (8) and the lens zone formed on it during the second stage (7), casts the photocurable material with the refraction index n3 and closes it with the half of the form that contains the diffractive structure in the form of rings, the radii of which coincide with the radii of the Fresnel zones. The material is photocured by UV light, the two halves of the casting mould are divided in such away that all the resultant components - zone (8), zone (7), zone (6) - stay on that half of the mould, which was used during the first stage, the uncured material is removed from the surface (2) of the resultant lens with the help of an appropriate solvent - isopropyl alcohol, for instance, - and is dried till the solvent is gone.

After that the resultant lens goes through additional UV treatment, then the resultant lens is placed into a closed container with isopropyl alcohol at the temperature of no lower than - 20°C and is held there for no longer than 24 hours, then it goes through thermal vacuum drying at the temperature no higher than 70°C for no longer than 6 hours.

The elements of the lens support can be formed during any one of the three stages of making the lens, both from the corresponding zone material (6, 7, 8) with the refraction index n1, n2, n3, respectively (as a monolith), and from different-materials (for example, polymethylmethacrylate or polypropylene).

This method makes it possible to produce thin multifocal lenses that provide for high visual function.

## Claims

1. A multifocal intraocular lens which comprises two external refractive surfaces and a longitudinal axis, with a diffractive structure superimposed on one of them in the form of the Fresnel zone, **characterized in that** between its external refractive surfaces at least one additional refractive surface is inserted, that divide the lens volume into zones manufactured from materials having different refraction coefficients.

2. A multifocal intraocular lens according to claim 1, **characterized in that** between the two external refractive surfaces exactly one additional refractive surface is inserted that divides the lens volume into two zones manufactured from two different kinds of materials having different refraction coefficients.

3. A multifocal intraocular lens according to claim 1, **characterized in that** between the two external refractive surfaces exactly two additional refractive surfaces are inserted that divide the lens volume into three zones manufactured from three different kinds of materials having different refraction coefficients.

4. A multifocal intraocular lens according to any one of the preceding claims, **characterized in that** the difference between the refraction coefficients of different kinds of materials used in manufacturing of the different zones of the lens should be equal or not less than 0.02.

5. A multifocal intraocular lens according to any one of the preceding claims, **characterized in that** all of the different zones are manufactured from materials which have refraction coefficients higher than the ocular fluid refraction coefficient by no less than 0.02.

6. A multifocal intraocular lens according to any one of the preceding claims, **characterized in that** the diffractive structure in the form of the Fresnel zone is calculated and manufactured in such a way that the optical power of this structure is ensured within the interval of 8 to 12 dioptres.

7. A multifocal intraocular lens according to any one of the preceding claims, **characterized in that** the microrelief of the diffractive structure is made in the form of chiefly right-angled profile grooves for every only even or only odd zones with the depth hmax of

about $h_{max} = \dfrac{0{,}55}{2(n_1 - n)}$ micrometers, with a relative inaccuracy within 5%, where n1 is the refraction index of the lens zone, which has the diffractive structure on it, n is the refraction index of the ocular fluid.

8. A multifocal intraocular lens according to any of the claims from 1 to 5, **characterized in that** the microrelief of the diffractive structure is made in the form of chiefly triangle profile grooves uniting every two neighboring Fresnel zones with the height hmax of the triangle of about $h_{max} = \dfrac{0{,}55}{(n_1 - n)}$ micrometers, with a relative inaccuracy within 5%, where n1 is the refraction index of the lens zone, which has the diffractive structure on it, n is the refraction index of the ocular fluid.

9. A multifocal intraocular lens according to any one of the preceding claims, **characterized in that** the Fresnel zone radii are directly proportional to the square roots of the integers designating the Fresnel zone's index number $r_k = r_1 \cdot \sqrt{k}$, where k = 1, 2, 3..., r1 is the radius of the first Fresnel zone calculated in such a way that the given optical power of the diffractive structure is ensured within the interval of 8 to 12 dioptres.

10. A multifocal intraocular lens according to any one of the preceding claims, **characterized in that** the Fresnel zone radii are made to be adapted to an eye allowing to reduce or minimize spherical aberrations of the cornea of the eye and of the external refractive surface of the lens.

11. A multifocal intraocular lens according to any one of the preceding claims, **characterized in that** one of the two external refractive surfaces is a sphere, and the other one is a plane with the diffractive structure in the form of the Fresnel zones superimposed all over its surface.

12. A multifocal intraocular lens according to claim 2, **characterized in that** the additional refractive surface in the central part is represented by a spherical segment with the diameter of $d_1$ 1.6 to 2.3 mm, further changing radially into a plane.

13. A multifocal intraocular lens according to claim 3, **characterized in that** the first additional refractive surface, counting from the external refractive surface with the diffractive structure, in the central part is represented by a spherical segment with the diameter $d_2$ 1.4 to 1.8 mm, further changing radially into a plane, and the second additional refractive surface in the central part is represented by a spherical segment with the diameter $d_3$ 2.3 to 2.6 mm, further changing radially into a plane, wherein $d_2 < d_3$.

**Patentansprüche**

1. Multifokale Intraokularlinse mit zwei externen refraktiven Flächen und einer Längsachse, wobei einer der Flächen eine diffraktive Struktur in Form der Fresnelzone überlagert ist, **dadurch gekennzeichnet, dass** zwischen ihren externen refraktiven Flächen zumindest eine weitere refraktive Fläche eingefügt ist, die das Linsenvolumen in Zonen unterteilt, die aus Materialien hergestellt sind, deren Refraktionskoeffizient verschieden ist.

2.  Multifokale Intraokularlinse gemäß Anspruch 1, **dadurch gekennzeichnet, dass** zwischen den beiden externen refraktiven Flächen exakt eine weitere refraktive Fläche eingefügt ist, die das Linsenvolumen in zwei Zonen unterteilt, die aus zwei unterschiedlich gearteten Materialien hergestellt sind, deren Refraktionskoeffizienten verschieden sind.

3.  Multifokale Intraokularlinse gemäß Anspruch 1, **dadurch gekennzeichnet, dass** zwischen den beiden externen refraktiven Flächen exakt zwei weitere refraktive Flächen eingefügt sind, die das Linsenvolumen in drei Zonen unterteilen, die aus drei unterschiedlich gearteten Materialien hergestellt sind, deren Refraktionskoeffizienten verschieden sind.

4.  Multifokale Intraokularlinse gemäß einem der vorhergehenden Ansprüche, **dadurch gekennzeichnet, dass** die Differenz zwischen den Refraktionskoeffizienten der verschiedenen Arten der Materialien, die bei der Herstellung der verschiedenen Zonen der Linse verwendet werden, gleich oder nicht kleiner als 0,02 sein sollten.

5.  Multifokale Intraokularlinse gemäß einem der vorhergehenden Ansprüche, **dadurch gekennzeichnet, dass** alle der verschiedenen Zonen aus Materialien hergestellt sind, deren Refraktionskoeffizienten um nicht weniger als 0,02 höher sind als der Refraktionskoeffizient des Okularfluids.

6.  Multifokale Intraokularlinse nach einem der vorhergehenden Ansprüche, **dadurch gekennzeichnet, dass** die refraktive Struktur in Form der Frensnelzone derart berechnet und hergestellt wird, dass der Brechwert dieser Struktur innerhalb des Zwischenabstands von 8 bis 12 Dioptrien sichergestellt ist.

7.  Multifokale Intraokularlinse nach einem der vorhergehenden Ansprüche, **dadurch gekennzeichnet, dass** das Mikrorelief der diffraktiven Struktur in Form von hauptsächlich rechtwinkligen Profilnuten für jede nur gerade oder nur ungerade Zone mit der Tiefe hmax von etwa

$$h_{\max} = \frac{0,55}{2(n_1 - n)}$$

Mikrometer bei einer relativen Ungenauigkeit von innerhalb 5% hergestellt wird, wobei n1 der Refraktionsindex der Linsenzone ist, welche die diffraktive Struktur trägt, und n der Refraktionsindex des Okularfluids ist.

8.  Multifokale Intraokularlinse nach einem der Ansprüche 1 bis 5, **dadurch gekennzeichnet, dass** das Mikrorelief der diffraktiven Struktur in Form von hauptsächlich dreieckigen Profilnuten hergestellt wird, die immer zwei benachbarte Fresnelzonen mit der Höhe hmax des Dreiecks von etwa

$$h_{\max} = \frac{0,55}{(n_1 - n)}$$

Mikrometer bei einer relativen Ungenauigkeit von innerhalb 5% vereinen, wobei n1 der Refraktionsindex der Linsenzone ist, welche die diffraktive Struktur trägt, und n der Refraktionsindex des Okularfluids ist.

9.  Multifokale Intraokularlinse nach einem der vorhergehenden Ansprüche, **dadurch gekennzeichnet, dass** die Fresnelzonenradii direkt proportional zu den Quadratwurzeln der Ganzzahlen sind, die die Indexzahl

$$r_k = r_1 \cdot \sqrt{k}$$

angeben, wobei k = 1, 2, 3 ..., r1 der Radius der ersten Fresnelzone ist, die derart berechnet wird, dass die gegebene Brechkraft der diffraktiven Struktur innerhalb eines Zwischenabstands von 8 bis 12 Dioptrien sichergestellt ist.

10. Multifokale Intraokularlinse nach einem der vorhergehenden Ansprüche, **dadurch gekennzeichnet, dass** eine Anpassung der Fresnelzonenradii an ein Auge bewirkt wird, wodurch es möglich ist, die sphärische Aberration der Kornea des Auges und der externen refraktiven Fläche der Linse zu reduzieren oder zu minimieren.

11. Multifokale Intraokularlinse nach einem der vorhergehenden Ansprüche, **dadurch gekennzeichnet, dass** eine der beiden externen refraktiven Flächen eine Kugelfläche und die andere eine Ebene ist, wobei die diffraktive Struktur in Form der Frensnelzonen ihrer gesamten Oberfläche überlagert ist.

12. Multifokale Intraokularlinse nach Anspruch 2, **dadurch gekennzeichnet, dass** die weitere refraktive Fläche in dem zentralen Teil durch ein sphärisches Segment mit dem Durchmesser d$_1$ von 1,6 bis 2,3 mm gebildet wird, das sich radial in einer Ebene fortsetzt.

13. Multifokale Intraokularlinse nach Anspruch 3, **dadurch gekennzeichnet, dass** die erste weitere refraktive Fläche, gerechnet ab der externen refraktiven Fläche mit der diffraktiven Struktur, in dem zentralen Teil durch ein sphärisches Segment mit dem Durchmesser $d_2$ von 1,4 bis 1,8 mm gebildet wird, das sich radial in einer Ebene fortsetzt, und dass sie zweite weitere refraktive Fläche in dem zentralen Teil durch ein sphärisches Segment mit dem Durchmesser $d_3$ von 2,3 bis 2,6 mm gebildet wird, das sich radial in einer Ebene fortsetzt, wobei $d_2 < d_3$ ist.

## Revendications

1. Lentille intraoculaire multifocale comportant deux surfaces réfractives externes et un axe longitudinal, avec d'une structure diffractive superposée à une d'eux sous forme de zone Fresnel, **caractérisée en ce qu'**il est insérée entre ses surfaces réfractives externes au moins une surface réfractive additionnelle qui divise le volume de la lentille en zones qui sont composées de matériaux ayant de coefficients de réfraction différents.

2. Lentille intraoculaire multifocale selon la revendication 1, **caractérisée en ce qu'**il est insérée entre les deux surfaces réfractives externes exactement une surface réfractive additionnelle qui divise le volume de la lentille en deux zones qui sont composées de deux sortes de matériaux ayant de coefficients de réfraction différents.

3. Lentille intraoculaire multifocale selon la revendication 1, **caractérisée en ce qu'**il sont insérées entre les deux surfaces réfractives externes exactement deux surfaces réfractives additionnelles qui divisent le volume de la lentille en trois zones qui sont composées de trois sortes de matériaux ayant de coefficients de réfraction différents.

4. Lentille intraoculaire multifocale selon l'une des revendications précédentes, **caractérisée en ce que** la différence entre les coefficients de réfraction de sortes différentes de matériaux utilisés dans la production des zones différentes de la lentille devait être égale ou pas inférieur à 0,02.

5. Lentille intraoculaire multifocale selon l'une des revendications précédentes, **caractérisée en ce que** tous les zones différentes sont composées de matériaux ayant de coefficients de réfraction qui ne sont pas supérieurs au coefficient de réfraction du fluide oculaire de moins que 0,02.

6. Lentille intraoculaire multifocale selon l'une des revendications précédentes, **caractérisée en ce que** la structure diffractive sous forme de zone Fresnel est calculée et faite de telle façon que la réfringence de cette structure est assurée dans un intervalle de 8 à 12 dioptries.

7. Lentille intraoculaire multifocale selon l'une des revendications précédentes, **caractérisée en ce que** le microrelief de la structure diffractive est fait en forme de rainures profilées essentiellement à angle droit pour chaque zone seulement paire ou seulement impair avec une profondeur hmax de

$$h_{\max} = \frac{0,55}{2(n_1 - n)}$$

micromètres environ, avec une inexactitude relative de 5% environ, n1 étant l'indice de réfraction de la zone de lentille qui porte la structure diffractive et n étant l'indice de réfraction du fluide oculaire.

8. Lentille intraoculaire multifocale selon l'une des revendications 1 à 5, **caractérisée en ce que** le microrelief de la structure diffractive est fait en forme de rainures profilées essentiellement triangulaires unissant deux zones Fresnel respectivement adjacentes avec une hauteur hmax du triangle de

$$h_{\max} = \frac{0,55}{(n_1 - n)}$$

micromètres environ, avec une inexactitude relative de 5% environ, n1 étant l'indice de réfraction de la zone de lentille qui porte la structure diffractive et n étant l'indice de réfraction du fluide oculaire.

9. Lentille intraoculaire multifocale selon l'une des revendications précédentes, **caractérisée en ce que** les rayons de la zone Fresnel sont directement proportionnels à la racine carrée des entiers désignant le nombre d'indice de la zone Fresnel

$$r_k = r_1 \cdot \sqrt{k},$$

k = 1, 2, 3 ..., r1 étant le rayon de la première zone Fresnel qui est calculée de cette façon que la réfringence donnée de la structure diffractive est assurée dans un intervalle de 8 à 12 dioptries.

10. Lentille intraoculaire multifocale selon l'une des revendications précédentes, **caractérisée en ce que** les rayons de la zone Fresnel sont déterminés à être adaptées à un oeil en permettant de réduire ou rendre minimale des aberrations sphériques de la cornée de l'oeil et de la surface réfractive externe de la lentille.

**11.** Lentille intraoculaire multifocale selon l'une des revendications précédentes, **caractérisée en ce que** l'une de deux surfaces réfractives externes est une sphère et l'autre est un plan portant la structure diffractive sous forme de zones Fresnel superposée sur toute sa surface.

**12.** Lentille intraoculaire multifocale selon la revendication 2, **caractérisée en ce que** la surface réfractive additionnelle dans la partie centrale est représentée par un segment sphérique ayant le diamètre $d_1$ de 1,6 à 2,3 mm, qui se change par la suite radialement en un plan.

**13.** Lentille intraoculaire multifocale selon la revendication 3, **caractérisée en ce que** la première surface réfractive additionnelle, comptée de la surface réfractive externe portant la structure diffractive, dans la partie centrale est représentée par un segment sphérique ayant le diamètre $d_2$ de 1,4 bis 1,8 mm, qui se change par la suite radialement en un plan, et la seconde surface réfractive additionnelle dans la partie centrale est représentée par un segment sphérique ayant le diamètre $d_3$ de 2,3 à 2,6 mm, qui se change par la suite radialement en un plan, avec $d_2 < d_3$.

Fig. 1

Fig. 2

Fig. 3

Fig. 4

Fig. 5

Fig. 6

# Multifocal Intraocular Lens

Axial intensity distribution (relative units)

Fig. 7

$\varnothing\, \mathsf{d}_1$

Fig. 8

$\varnothing\, \mathsf{d}_2$

$\varnothing\, \mathsf{d}_3$

6

7

8

Fig. 9

13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4637697 A
- WO 2004113959 A
- US 5089023 A
- US 7025456 B
- RU 2303961
- US 5344447 A